(19) Europäisches Patentamt
European Patent Office
Office européen des brevets

(11) **EP 2 781 192 A1**

(12) **EUROPEAN PATENT APPLICATION**
published in accordance with Art. 153(4) EPC

(43) Date of publication:
**24.09.2014 Bulletin 2014/39**

(51) Int Cl.:
*A61B 8/08* *(2006.01)*

(21) Application number: **12849692.4**

(86) International application number:
**PCT/JP2012/075746**

(22) Date of filing: **04.10.2012**

(87) International publication number:
**WO 2013/073304 (23.05.2013 Gazette 2013/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.11.2011 JP 2011251740**

(71) Applicant: **Hitachi Aloka Medical, Ltd.**
**Tokyo 181-8622 (JP)**

(72) Inventor: **WAKI, Koji**
**Mitaka-shi**
**Tokyo 181-8622 (JP)**

(74) Representative: **Strehl Schübel-Hopf & Partner**
**Maximilianstrasse 54**
**80538 München (DE)**

(54) **ULTRASONIC DIAGNOSTIC DEVICE AND ULTRASONIC IMAGE GENERATION METHOD**

(57) Provided is an ultrasonic diagnostic device that improves the frame rate (or volume rate), is resistant to movement, and is capable of constructing ultrasonic images at a high frame rate or a high volume rate. The ultrasonic diagnostic device comprises: a plurality of transducers arranged on an ultrasonic probe, that oscillate at the same time, and irradiate an ultrasonic beam on a subject; and an ultrasonic image generation unit that generates a first ultrasonic data as a result of the plurality of transducers oscillating at the same time and irradiating a first ultrasonic beam on the subject, generates a second ultrasonic data as a result of the plurality of transducers oscillating at the same time and irradiating a second ultrasonic beam on the subject, and generates an acoustic image on the basis of the first ultrasonic data and the second ultrasonic data.

F I G . 1

EP 2 781 192 A1

**Description**

Technical Field

The present invention relates to an ultrasonic diagnostic device, and more particularly, to an ultrasonic diagnostic device that constructs a 4D ultrasonic image.

Background Art

A conventional ultrasonic diagnostic device employs an ultrasonic probe to transmit ultrasonic waves to the inside of a subject, receives the reflected echo signal of the ultrasonic wave in consonance with the living tissue structure of the subject, and provides a tomographic monochrome (black and white) image for the cross section of the subject.
Further, the conventional ultrasonic diagnostic device mechanically reciprocates the ultrasonic probe to compare two image frames obtained at different times in the same section, and calculates the displacement of the living tissue, and thereafter, provides an elastographic image for elastographic information (e.g., hardness information and strain information) of the tissue. Furthermore, the conventional ultrasonic diagnostic device alternately scans the tomographic image and the elastographic image, and superimposes the tomographic image with the elastographic image to be displayed (see, for example, patent document 1).
Moreover, for providing a three-dimensional elastographic image, the conventional ultrasonic diagnostic device selects a plurality of elastographic images generated in the state wherein the same pressure is applied to the tissue of a subject, and synthesizes these selected elastographic images to create a three-dimensional elastographic image, or employs only image frames that have large values of correlation coefficients to create a three-dimensional elastographic image (see, for example, patent document 2).
Further, the conventional ultrasonic diagnostic device displays a three-dimensional image (stereoscopic image) in real time to generate a 4D ultrasonic image.

Citation List

Patent Documents

PTL 1: Japanese Patent Laid-Open No. 2004-141505
PTL 2: Japanese Patent Laid-Open No. 2008-259555

Summary of Invention

Technical Problem

However, the conventional ultrasonic diagnostic device is susceptible to movements (e. g. , camera shakes or body motions associated with pressure). Especially, since the location for obtaining an ultrasonic image is shifted when the ultrasonic probe mechanically reciprocates, or a time difference between the two image frames is increased depending on the period required for the reciprocal movement of the ultrasonic probe, the conventional ultrasonic diagnostic device encounters difficulty in employing a 4D system (an image generation system that updates a three-dimensional image, as needed, and displays the updated image in real time) to construct a motion-compensated 4D ultrasonic image. Furthermore, since the conventional ultrasonic diagnostic device generates an ultrasonic beam by sequentially vibrating, in the arrangement direction, a plurality of transducers that are arranged for the ultrasonic probe, an extended period of time is required for obtaining a tomographic image or an elastographic image, and difficulty is encountered in creating a 4D ultrasonic image at a high frame rate or at a high volume rate.
Therefore, one objective of the present invention is to provide an ultrasonic diagnostic device that can construct an ultrasonic image (e.g., a 4D ultrasonic image) at a high frame rate or a high volume rate.

Solution to Problem

An ultrasonic diagnostic device according to the present invention includes:

a plurality of transducers arranged in an ultrasonic probe so as to be vibrated at the same time for projecting an ultrasonic beam to a subject; and

an ultrasonic image generation unit for generating first ultrasonic data through projection of a first ultrasonic beam to the subject by vibrating the plurality of transducers at the same time, and generating second ultrasonic data through projection of a second ultrasonic beam to a subject by vibrating the plurality of transducers at the same time, and for generating an elastographic image based on the first ultrasonic data and the second ultrasonic data.

According to this arrangement, since a plurality of transducers are vibrated at the same time to project an ultrasonic beam to the subject, a single performance of irradiation of the ultrasonic beam is required to generate ultrasonic data for the cross section of the subject, and therefore, the frame rate (or the volume rate) is increased, and the elastographic image can be constructed at a high frame rate or a high volume rate.

Advantageous Effects of Invention

According to the present invention, there is provided an ultrasonic diagnostic device that can improve the frame rate (or the volume rate) because a plurality of transducers are vibrated at the same time to project an ultrasonic beam to a subject, and therefore, ultrasonic data for the cross section of the subject are generated by a single performance of irradiation of the ultrasonic beam.

Brief Description of Drawings

FIG. 1 is a diagram illustrating an ultrasonic diagnostic device according to one embodiment of the present invention.
FIG. 2 is a diagram illustrating transmission and reception of a plane wave.
FIG. 3 is a diagram showing generation of volume data (ultrasonic data) through the reciprocal movement of an ultrasonic probe.
FIG. 4 is a diagram showing a case wherein a plurality of transducers are sequentially vibrated and a case wherein the transducers are vibrated at the same time.
FIG. 5 is a diagram for a comparison between a forward movement and a backward movement for a case wherein the plurality of transducers are sequentially vibrated and for a case wherein the transducers are vibrated at the same time.
FIG. 6 is a diagram for explaining the interval for performing correlation calculation for volume data.
FIG. 7 is a diagram for explaining sequential transmission and simultaneous transmission of the plurality of transducers in a frame data acquisition sequence.
FIG. 8 is a diagram for explaining alternate acquisition of a tomographic image (monochrome image), an elastographic image (elastography), and a Doppler image.
FIG. 9 is a diagram for explaining a case wherein an elastographic image is generated based on displacement in the directions in three-dimensional space, (x, y, z).

Description of Embodiment

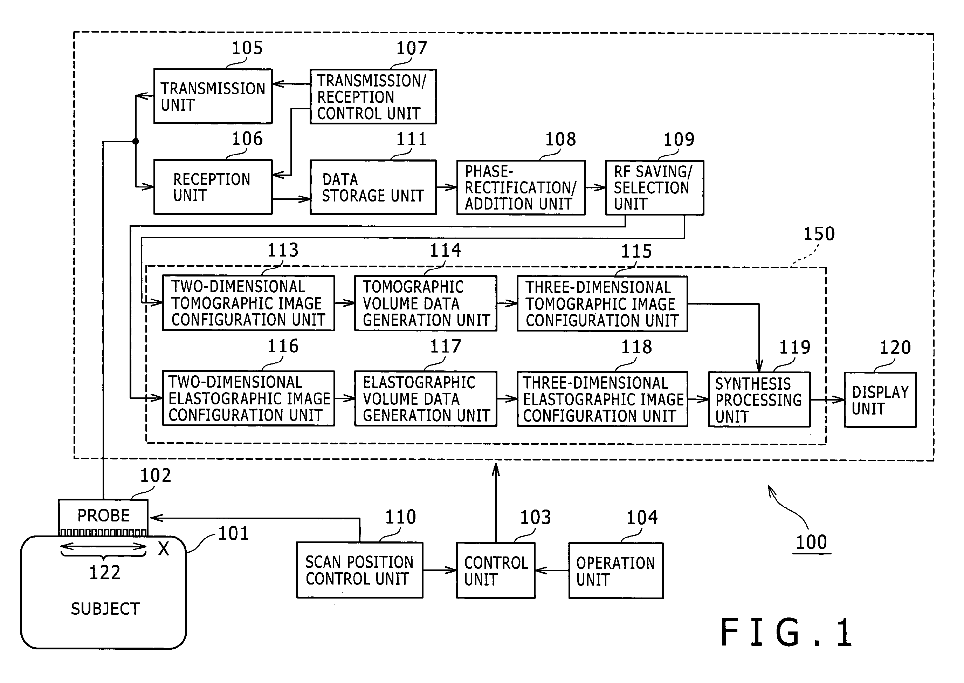

An ultrasonic diagnostic device for the embodiment of the present invention will now be described by reference to the drawings.
An ultrasonic diagnostic device 100 of the embodiment of this invention is shown in FIG. 1. As shown in FIG. 1, the ultrasonic diagnostic device 100 includes: an ultrasonic probe 102 that is employed in contact with a subject 101; a transmission unit 105 that repetitively transmits ultrasonic waves to the subject 101 via the ultrasonic probe 102 at predetermined time intervals; a reception unit 106 that receives a reflected echo signal reflected from the subject 101; a transmission/reception control unit 107 that controls the transmission unit 105 and the reception unit 106; a data storage unit 111 that temporarily stores the reflected echo received by the reception unit 106; a phase-rectification/addition unit 108 that performs phase-rectification and addition in order to form the individual beams received from the data storage unit 111; an RF saving/selection unit 109 that stores RF signal frame data (ultrasonic data) generated by the phase-rectification/addition unit 108; an ultrasonic image generation unit 150 that generates an ultrasonic image based on the RF signal frame data stored in the RF saving/selection unit 109; and a display unit 120 that displays an ultrasonic image generated by the ultrasonic image generation unit 150. The ultrasonic diagnostic device 100 also includes: a control unit 103 that controls the individual components; and an operation unit 104 that is employed to provide various entries for the control unit 103. The operation unit 104 includes a keyboard and a trackball.
A plurality of transducers 122 (vibrators) are arranged in the ultrasonic probe 102. The ultrasonic probe 102 has a function of transmitting ultrasonic waves to, and receiving the ultrasonic waves from the subject 101 through the plurality of transducers 122. Rectangular-shaped or fan-shaped transducers 122 are provided for the ultrasonic probe 102. The ultrasonic probe 102 is mechanically reciprocated in a direction (along a short axis) perpendicular to a direction x in which the plurality of transducers 122 are arranged. Since the plurality of transducers 122 are mechanically reciprocated by mechanically moving the ultrasonic probe 102, three-dimensional transmission and reception of an ultrasonic wave are performed.

The multiple transducers 122 arranged in the ultrasonic probe 102 are vibrated at the same time to project the ultrasonic beam to the subject 101. These multiple transducers 122 may also be vibrated sequentially in the arrangement direction x to project the ultrasonic beam to the subject 101.

The transmission unit 105 generates a transmission pulse, with which the plurality of transducers 122 of the ultrasonic probe 102 are to be driven to generate ultrasonic waves. The transmission unit 105 has a function for setting a predetermined depth for the convergence point of the ultrasonic wave that is transmitted, and a function for encouraging generation of a plane wave. The reception unit 106 employs a predetermined gain to amplify the reflected echo signal received by the ultrasonic probe 102, and generates an RF signal (i.e., a received signal). The ultrasonic transmission/reception control unit 107 controls the transmission unit 105 and the reception unit 106.

The phase-rectification/addition unit 108 controls the phase of the RF signal amplified by the reception unit 106, forms an ultrasonic beam with respect to one convergence point or a plurality of convergence points, and generates RF frame data (corresponding to RAW data).

The ultrasonic image generation unit 150 also includes a two-dimensional tomographic image configuration unit 113, a tomographic volume data generation unit 114, a three-dimensional tomographic image configuration unit 115, a two-dimensional elastographic image configuration unit 116, an elastographic volume data generation unit 117, a three-dimensional elastographic image configuration unit 118, and a synthesis processing unit 119.

The two-dimensional tomographic image configuration unit 113 generates a two-dimensional tomographic image (ultrasonic image) based on the RF signal frame data stored in the RF saving/selection unit 109. The tomographic volume data generation unit 114 employs the two-dimensional tomographic image obtained by the two-dimensional tomographic image configuration unit 113, performs three-dimensional coordinate transformation in accordance with the two-dimensional tomographic image acquisition location, and generates tomographic volume data (ultrasonic data). The three-dimensional tomographic image configuration unit 115 performs volume rendering based on the luminance and opacity of the tomographic volume data, and creates a three-dimensional tomographic image (ultrasonic image).

The two-dimensional elastographic image configuration unit 116 constructs a two-dimensional elastographic image (ultrasonic image) based on a plurality of sets of RF signal frame data stored in the RF saving/selection unit 109. The elastographic volume data generation unit 117 employs the two-dimensional elastographic image obtained by the two-dimensional elastographic image configuration unit 116, and performs the three-dimensional coordinate transformation in accordance with the two-dimensional elastographic image acquisition position and generates elastographic volume data (ultrasonic data). The three-dimensional elastographic image configuration unit 118 performs volume rendering based on the elasticity value and the opacity of the elastographic volume data, and creates a three-dimensional elastographic image (ultrasonic image).

The synthesis processing unit 119 synthesizes the two-dimensional tomographic image with the two-dimensional elastographic image, or synthesizes the three-dimensional tomographic image with the three-dimensional elastographic image. The display unit 120 displays, for example, a synthesis image obtained by the synthesis processing unit 119, or a two-dimensional tomographic image (ultrasonic image).

Next, a specific operation performed by the ultrasonic image generation unit 150 will be described. Based on the setting condition for the control unit 103, the two-dimensional tomographic image configuration unit 113 receives RF frame data output by the RF saving/selection unit 109, performs signal processing, such as gain correction, log compression, edge enhancement, and filtering, and constructs a two-dimensional tomographic image. The ultrasonic probe 102 can detect a transmission/reception direction $(\theta, \phi)$ at the same time as performing transmission/reception of the ultrasonic waves, and in accordance with the transmission/reception direction $(\theta, \phi)$ that corresponds to the acquisition location for a two-dimensional tomographic image, the tomographic volume data generation unit 114 performs the three-dimensional coordinate transformation based on a plurality of two-dimensional tomographic images, and generates tomographic volume data.

The three-dimensional tomographic image configuration unit 115 employs the tomographic volume data to form a three-dimensional tomographic image. The three-dimensional tomographic image configuration unit 115 performs volume rendering based on equations (1) to (3).

$$\text{Cout}(i) = \text{Cout}(i - 1) + (1 - \text{Aout}(i - 1)) \cdot A(i) \cdot C(i) \cdot S(i)$$

$$\cdots \cdots (1)$$

$$\mathrm{Aout(i)} = \mathrm{Aout(i - 1)} + (1 - \mathrm{Aout(i - 1))}\cdot \mathrm{A(i)}$$

$$\cdots \cdots (2)$$

$$\mathrm{A(i)} = \mathrm{Opacity[C(i)]} \qquad \cdots \cdots (3)$$

Here, C (i) is a luminance value of the i-th voxel located along the line of sight in a case wherein a three-dimensional tomographic image is viewed from a predetermined point on a two-dimensional projection plane that is created. Cout(i) is a pixel value to be output. For example, when the luminance values of N voxels are arranged along the line of sight, luminance value Cout (N-1) obtained by adding I = 0 to (N - 1) is a final pixel value to be output. Cout (i-1) indicates a total value up to the (i-1)th voxel.

Further, A(i) indicates opacity for the i-th luminance value present on the line of sight, and as shown in equation (3), is provided as a tomographic opacity table ranging from a value of 0 to 1.0. When opacity is examined in the tomographic opacity table based on the luminance value, a rate of contribution to a two-dimensional projection plane (three-dimensional tomographic image) to be output is determined.

S (i) is a weight component for shading that is calculated by employing the luminance C (i) and the gradient obtained based on the peripheral pixel values, and indicates the shading enhancement effects; for example, in a case wherein the light source is aligned with the normal line of a plane where the voxel is located in the center, 1.0 is provided because light is reflected most strongly, whereas in a case wherein the light source is perpendicular to the normal line, 0.0 is provided.

For both Cout (i) and Aout (i), 0 is employed as the initial value. As represented by equation (2), Aout (i) is added each time a voxel is passed by, and converges to 1.0. Therefore, as represented by equation (1), in a case wherein the total value Aout (i-1) for opacity up to the (i-th) voxel has reached about 1.0, the luminance value C(i) for the i-th voxel and later is not incorporated for an output image.

The two-dimensional elastographic image configuration unit 116 measures displacement by employing a plurality of sets of RF signal frame data (ultrasonic data) stored in the RF saving/selection unit 109. Then, the two-dimensional elastographic image configuration unit 116 calculates a value of elasticity based on the obtained displacement, and forms a two-dimensional elastographic image. That is, a first ultrasonic beam is emitted to the subject 101 by vibrating the plurality of transducers 122 of the ultrasonic probe 102 at the same time, and the first ultrasonic data are generated, while a second ultrasonic beam is emitted to the subject 101 by vibrating the transducers 122 at the same time, and second ultrasonic data are generated, and the two-dimensional elastographic image configuration unit 116 compares the first ultrasonic data with the second ultrasonic data, calculates an elasticity value based on the displacement of the subject 101, and generates an elastographic image (ultrasonic image). The elasticity value includes at least one type of elastographic information, such as strain, elasticity, displacement, viscosity, stiffness, or a strain ratio.

The elastographic volume data generation unit 117 performs the three-dimensional transformation for a plurality of two-dimensional elastographic images in accordance with the transmission/reception direction (θ, φ) that corresponds to the acquisition position for a two-dimensional elastographic image, and generates elastographic volume data (ultrasonic data). The three-dimensional elastographic image configuration unit 118 employs the elasticity value to perform volume rendering for the elastographic volume data (ultrasonic data), and forms a three-dimensional elastographic image (ultrasonic image).

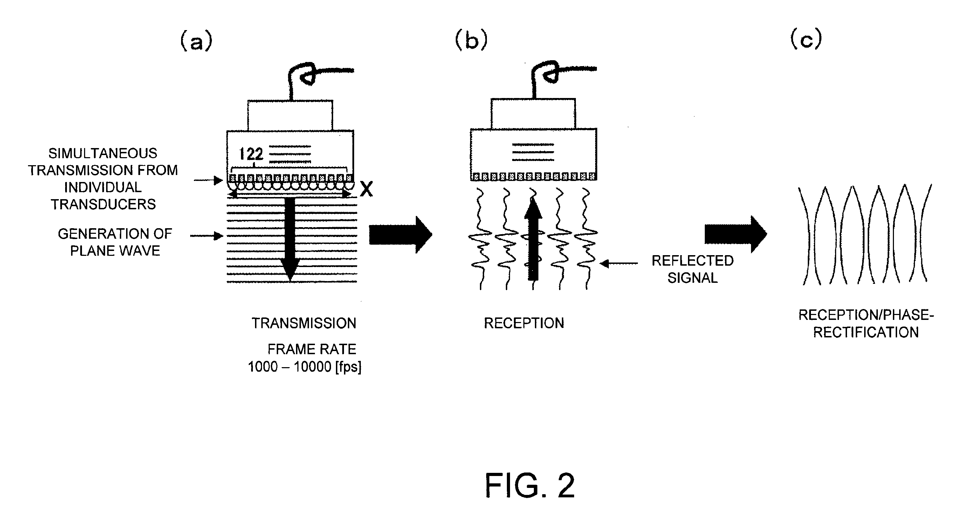

An explanation will now be given for a case wherein the plurality of transducers 122 are vibrated at the same time to generate an ultrasonic beam that is a plane wave. FIG. 2 is a diagram illustrating transmission and reception of a plane wave. FIG. 2(a) is a diagram illustrating transmission of a plane wave. When sound waves with almost the same intensity, for example, are generated by the individual transducers 122 of the ultrasonic probe 122, a plane wave parallel to the surface of the ultrasonic probe 102 can be obtained. Since the plane wave (ultrasonic beam) is emitted to the subject 101 by vibrating the plurality of transducers 122 at the same time, RF frame data (ultrasonic data) for the cross section of the subject 101 are generated through a single performance of irradiation of the plane wave (ultrasonic beam), and therefore, as compared with an ultrasonic diagnostic device that sequentially vibrates a plurality of transducers in the arrangement direction x to emit the ultrasonic beam to the subject 101, the frame rate (or the volume rate) is improved, and a frame rate of about 1000 to 10000 [fps] is obtained.

FIG. 2 (b) is a diagram illustrating reception of a plane wave. As shown in FIG. 2(b), since the individual transducers 122 receive a reflected signal in the period of open time, a reflected signal reflected from the tissue of the subject 101 is obtained at each temporal position in an imaging area. Data for the reflected signal in the imaging area are stored in the data storage unit 111. As illustrated in FIG. 2(c), since phase-rectification is performed for the reflected signal data that have been received, a plurality of beam lines can be formed inside the imaging area. The signal reception and phasing are performed in the manner that, on the assumption that sounds generated at a predetermined location will spread on the spherical surface, the phase-rectification/addition unit 108 corrects a time delay with which the signal reaches the individual transducers due to the spread of the sound.

As described above, the plurality of transducers 122 are arranged for the ultrasonic probe 102, and are vibrated at the same time to emit the ultrasonic beam to the subject 101. The ultrasonic image generation unit 150 generates a three-dimensional ultrasonic image (a three-dimensional tomographic image, a three-dimensional elastographic image, etc.) based on the ultrasonic data obtained when the ultrasonic probe 102 is moved.

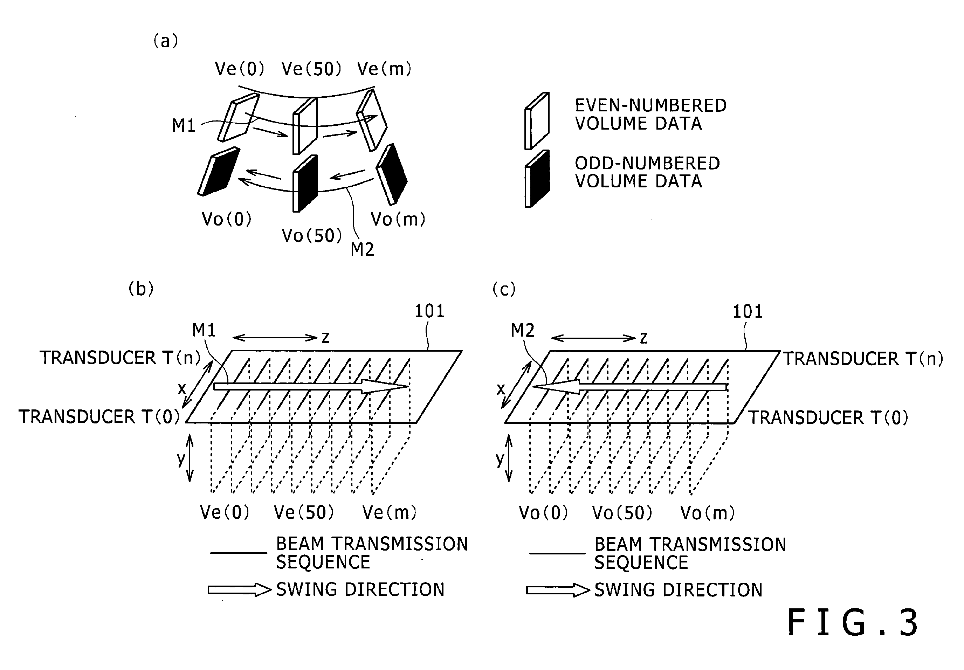

Next, an explanation will be given for the acquisition of an ultrasonic image (e.g., a 4D elastographic image) by employing the ultrasonic probe 102 (e.g., a 4D probe) that is mechanically driven. FIG. 3 is a diagram showing generation of volume data (ultrasonic data) by the reciprocal movement of the ultrasonic probe 102. As shown in FIG. 3(a), first, the zeroth (even numbered) even-numbered volume data (the first ultrasonic data) generated by a forward movement M1 of the ultrasonic probe 102. Then, the first (odd numbered) odd-numbered volume data (the second ultrasonic data) are generated by a backward movement M2 of the ultrasonic probe 102. Thereafter, the second (even numbered) even-numbered volume data are generated by the forward movement M1 of the ultrasonic probe 102. Through the repetition of the forward and backward movements M1 and M2 of the ultrasonic probe 102, the even-numbered volume data (first ultrasonic data) and the odd-numbered volume data (second ultrasonic data) are generated.

The tomographic volume data generation unit 114 employs the acquisition positions for two-dimensional tomographic images (ultrasonic images) Ve(0)~Ve(50)~Ve(m) and Vo(0)~Vo(50)~Vo(m), generated by the two-dimensional tomographic image configuration unit 113, and performs the three-dimensional coordinate transformation for the two-dimensional tomographic images and generates tomographic volume data (ultrasonic data), so that the even-numbered volume data (first ultrasonic data) and the odd numbered volume data (second ultrasonic data) are obtained.

FIGs. 3(b) and (c) are diagrams showing the irradiation of the ultrasonic beam by vibrating the plurality of transducers 122 at the same time in accordance with the movement of the ultrasonic probe 102. As shown in FIGs. 3(b) and (c), since the ultrasonic probe 102 mechanically reciprocates in a direction z (along the short axis) perpendicular to a direction x in which a plurality of transducers T(0) to T (n) are arranged, a scanning sequence is performed by the forward and backward movements M1 and M2 of the ultrasonic probe 102. The scanning sequence for the forward swing (forward movement) is shown in FIG. 3(b), while the scanning sequence for the backward swing (backward movement) is shown in FIG. 3(c).

Specifically, provided are: a plurality of transducers, which are vibrated at the same time to project an ultrasonic beam to a subject; and the ultrasonic image generation unit 150 that generates the first ultrasonic data through projection of the first ultrasonic beam to the subject 101 by vibrating the plurality of transducers at the same time, and generates the second ultrasonic data through projection of the ultrasonic beam to the subject by vibrating the plurality of transducers at the same time, and that employs the first ultrasonic data and the second ultrasonic data to generate an elastographic image. Furthermore, the first ultrasonic data are generated through projection of the ultrasonic beam to the subject 101 by vibrating, at the same time, the plurality of transducers arranged for the ultrasonic probe 102, while the second ultrasonic data are generated through projection of the ultrasonic beam to the subject 101 by vibrating the plurality of transducers at the same time, and the first ultrasonic data and the second ultrasonic data are employed to generate an ultrasonic image.

In this case, since the plurality of transducers 122 are vibrated at the same time to perform irradiation of the ultrasonic beam, only a single performance of irradiation of a plane wave is required to generate RF signal frame data (ultrasonic data) for the cross section of the subject 101, and therefore, as compared with the ultrasonic diagnostic device that sequentially vibrates the plurality of transducers in the arrangement direction x to emit the ultrasonic beam to the subject 101, the frame rate (or the volume rate) is increased. When the frame rate (or the volume rate) is improved, a motion-compensated 4D ultrasonic image (4D elastographic image) can be constructed by a 4D system.

The plurality of transducers of the ultrasonic probe 102 perform reciprocal movements, and the ultrasonic image generation unit 150 generates an elastographic image based on the first ultrasonic data, generated during the forward movement, and the second ultrasonic data generated during the backward movement, performed immediately after the forward movement. According to this arrangement, since an elastographic image can be generated based on the ultrasonic data obtained during the forward movement and the backward movement, which is performed immediately after the forward movement, the interval of the correlation operation can be reduced, and a motion-compensated ultrasonic image (e.g., a 4D elastographic image) can be constructed.

There are provided a plurality of transducers, which are arranged for the ultrasonic probe 102 and are vibrated at the same time to emit the ultrasonic beam to the subject 101, and the ultrasonic image generation unit 150, which generates a three-dimensional ultrasonic image based on the ultrasonic data that are obtained when the plurality of transducers are moved.

According to this arrangement, since the plurality of transducers are vibrated at the same time and project the ultrasonic beam to the subject 101, ultrasonic data for the cross section of the subject 101 are generated through a single performance of irradiation of the ultrasonic beam, and therefore, the frame rate (or the volume rate) is increased, and a motion-compensated ultrasonic image (a tomographic image, an elastographic image, a Doppler image, etc.) can be constructed at a high frame rate, or a high volume rate.

Furthermore, the plane wave is generated by the plurality of transducers. According to this arrangement, when the plurality of transducers are vibrated at the same time and emit the ultrasonic beam to the subject 101, the plane wave can be generated, and ultrasonic data for the cross section of the subject 101 can be generated by a single performance of irradiation of the ultrasonic beam.

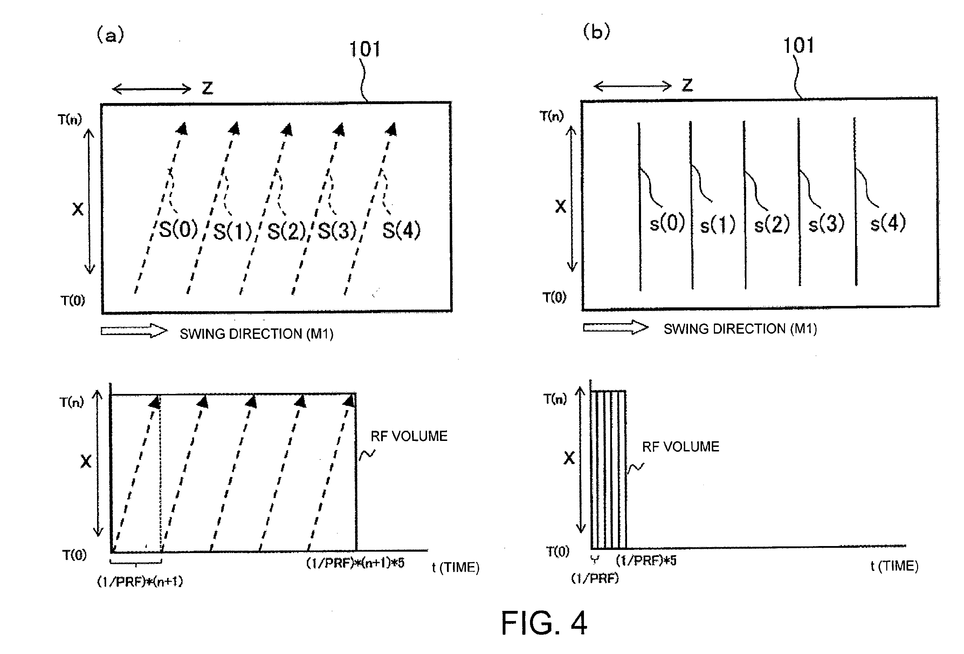

FIG. 4 is a diagram showing a case wherein the ultrasonic beam is emitted by sequentially vibrating the plurality of transducers 122, and a case wherein the ultrasonic beam is emitted by vibrating the plurality of transducers 122 at the same time. As shown in FIG. 4(a), during the forward movement M1, the ultrasonic probe 102 is shifted in the short axial direction z. As the ultrasonic probe 102 is shifted in the short axial direction z, the plurality of transducers T(0) to T(n) arranged in the arrangement direction x are vibrated sequentially. When a repetition frequency for pulses transmitted by the transducers is denoted by PRF (Pulse Repetition Frequency), the period of the PRF (pulse repetition period) is 1/PRF = PRT (Pulse Repetition Time). That is, a period of "1/PRF" is required for vibrating a single transducer to transmit a pulse.

Therefore, a period of " (1/PRF) * (n + 1)" is required for the plurality of transducers T(0) to T(n) to be sequentially vibrated and transmit pulses for generating RF signal frame data for one cross section (e.g., cross section S(0)). Furthermore, a period of " (1/PRF) * (n + 1) * (m + 1) " is required for the scanning sequence to obtain two-dimensional tomographic images (ultrasonic images) Ve(0)~Ve(m) for a plurality of cross sections S(0) to S(m). Referring to FIG. 4(a), a period of "(1/PRF) * (n + 1)*5" is required for obtaining two-dimensional tomographic images (ultrasonic images) Ve(0) to Ve(4) for a plurality of cross sections S(0) to S(4).

Meanwhile, as shown in FIG. 4(b), during the backward movement M2, the ultrasonic probe 102 is shifted in the short axial direction z, and the plurality of transducers T (0) to T (n) arranged in the arrangement direction x are vibrated at the same time. In this case, the plurality of transducers T(0) to T (n) are vibrated at the same time and transmit pulses, and RF signal frame data (ultrasonic data) for a cross section (e.g., cross section s(0)) are generated through a single performance of irradiation of a plane wave (ultrasonic beam). Therefore, a period of "1/PRF" is required for generation of RF signal frame data for a single cross section (e.g., cross section s(0)). Further, a period of "(1/PRF)*(m + 1)" is required for the scanning sequence to obtain two-dimensional tomographic images (ultrasonic images) Ve(0) to Ve(m) for the plurality of cross sections S(0) to S(m). Referring to FIG. 4(b), a period of "(1/PRF)*5" is required to obtain two-dimensional tomographic images (ultrasonic images) Ve(0) to Ve(4) for the plurality of cross sections s(0) to s(4).

As described above, in a case wherein the plurality of transducers 122 are vibrated at the same time, the frame rate (or the volume rate) is increased by (n + 1) times, as compared with the case wherein the transducers are vibrated sequentially.

Referring to FIGs. 4 to 6, an explanation will now be given for a case wherein generation of an elastographic image is performed based on a correlation coefficient of ultrasonic data. The ultrasonic probe 102 performs the forward and backward movements M1 and M2. The ultrasonic image generation unit 150 generates an elastographic image based on the coefficient of a correlation between the first ultrasonic data (even-numbered volume data), generated during the forward movement M1 of the ultrasonic probe 102, and the second ultrasonic data (odd-numbered volume data), generated during the backward movement M2 performed immediately after the forward movement M1.

As shown in FIG. 4, in the scanning sequence, the ultrasonic probe 102 is moved in the swing direction (the short axial direction z) at a constant velocity. Therefore, in a case as shown in FIG. 4(a), wherein the plurality of transducers 122 are sequentially vibrated to emit the ultrasonic beam, the ultrasonic beam is projected to the cross sections S (0) to S (4) located oblique to the direction x in which the plurality of transducers 122 are arranged.

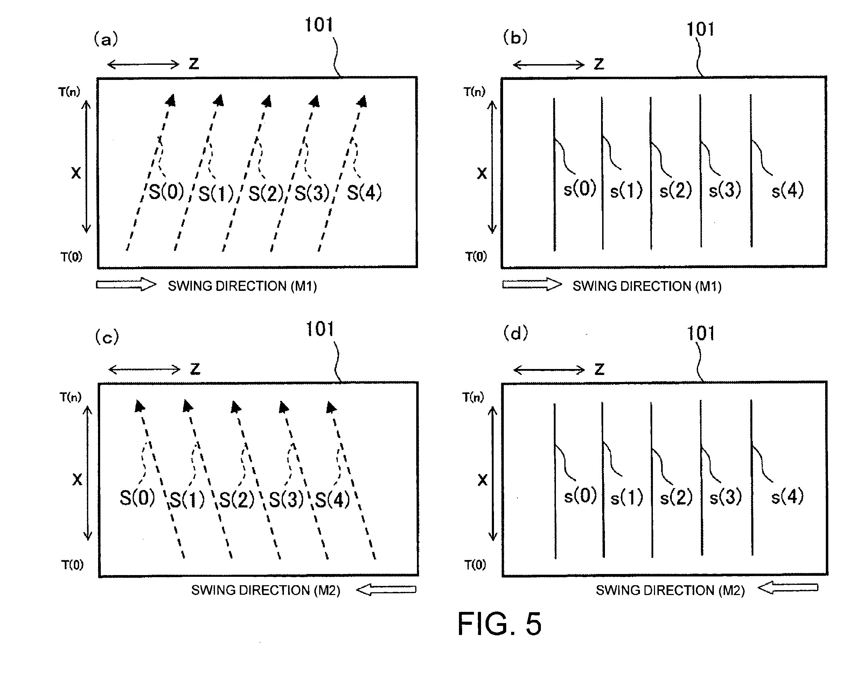

FIG. 4 (a) is a diagram for explaining the forward movement M1 of the ultrasonic probe 102. During the forward movement M1, since the plurality of transducers 122 are sequentially vibrated as shown in FIG. 4(a), the ultrasonic beam is emitted to the cross sections S(0) to S(4) that are inclined in the swing direction (the direction along the forward movement M1). Meanwhile, in the case of the backward movement M2 of the ultrasonic probe 102, since during the backward movement M2 the plurality of transducers 122 are sequentially vibrated as shown in FIG. 5(c), the ultrasonic beam is emitted to the cross sections S(0) to S(4) inclined in the swing direction (the direction along the backward movement M2).

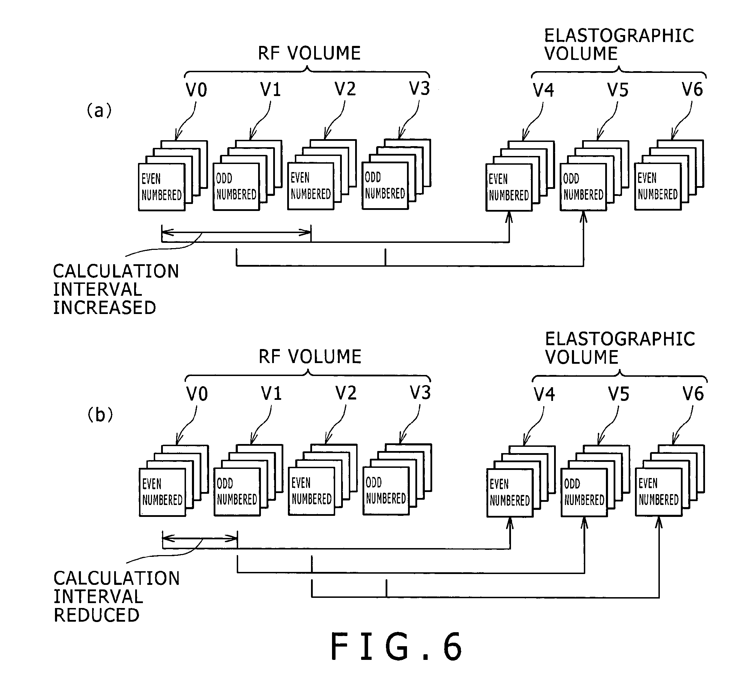

Therefore, as shown in FIGs. 5 (a) and (c), since the angle of the cross section S relative to the arrangement direction x during the backward movement M2 is different from the angle during the forward direction M1, the acquisition location for the ultrasonic image is deviated. That is, the ultrasonic image acquisition location for the forward movement M1 and the ultrasonic image acquisition location for the backward movement M2 differ from each other. Therefore, in a case as shown in FIG. 6(a), wherein the plurality of transducers 122 are sequentially vibrated to emit the ultrasonic beam, a plurality of even-numbered volume data sets V0 and V2 (RF volume data), generated during the forward movement M1, are compared with each other, instead of comparing the even-numbered volume data V0 (the first ultrasonic data), generated during the forward movement M1, with the odd-numbered volume data V1 (the second ultrasonic data) generated during the backward movement M2, and the resultant correlation coefficient is employed to generate an elastographic image V4 (elastographic volume image).

Further, a plurality of odd-numbered volume data sets V1 and V3 (RF volume data), generated during the backward movement M2, are compared with each other, and the correlation coefficient is employed to generate an elastographic image V5 (elastographic volume image).

On the other hand, in a case as shown in FIGs. 5(b) and (d), wherein the plurality of transducers 122 are vibrated at the same time to emit the ultrasonic beam, the angle of the cross section S is constant relative to the arrangement direction x, and the acquisition location for an ultrasonic image is not deviated, so that the ultrasonic image acquisition position for the forward movement M1 is the same as the ultrasonic image acquisition position for the backward movement M2. Therefore, as shown in FIG. 6(b), when the even-numbered volume data V0 (the first ultrasonic data) generated during the forward movement M1 are compared with the odd-numbered volume data V1 (the second ultrasonic data) generated during the backward movement M2, an elastographic image V4 (elastographic volume image) is generated based on the correlation coefficient. Further, when the odd-numbered volume data V1 (RF volume data) are compared with the even-numbered volume data V2 (RF volume data), an elastographic image V5 (elastographic volume image) is generated based on the correlation coefficient. Furthermore, when the even-numbered volume data V2 (RF volume data) are compared with odd-numbered volume data V3 (RF volume data), an elastographic image V6 (elastographic volume image) is generated based on the correlation coefficient.

In this manner, the ultrasonic image generation unit 150 generates an elastographic image based on the coefficient of correlation between the first ultrasonic data (even-numbered volume data), generated during the forward movement M1 of the ultrasonic probe 102, and the second ultrasonic data (odd-numbered volume data) generated during the backward movement M2, performed immediately after the forward movement M1.

The plurality of transducers of the ultrasonic probe 102 may perform reciprocal movements, and the ultrasonic image generation unit 150 may generate an elastographic image based on the first ultrasonic data, generated during the forward movement or the backward movement, and the second ultrasonic data generated during the movement that is the same as the forward or the backward movement.

For example, the ultrasonic image generation unit 150 may employ the first ultrasonic data, generated during the forward movement, and the second ultrasonic data generated also during the forward movement to construct an elastographic image. Further, the ultrasonic image generation unit 150 may employ the first ultrasonic data, generated during the backward movement, and the second ultrasonic data generated also during the backward movement to construct an elastographic image. The second ultrasonic data are ultrasonic data generated after the first ultrasonic data by irradiation of the ultrasonic beam during the forward movement or the backward movement. That is, the first ultrasonic data and the second ultrasonic data are ultrasonic data for the cross sections adjacent to each other. When the first ultrasonic data are represented by Ve(n), the second ultrasonic data are Ve(n + 1).

In a case as shown in FIG. 6, wherein the plurality of transducers 122 are sequentially vibrated to emit the ultrasonic beam, a correlation calculation is performed across a set of RF volume data, and therefore, the calculation interval is extended.

On the other hand, in a case wherein the plurality of transducers 122 are vibrated at the same time to emit the ultrasonic beam, the correlation calculation is performed for RF volume data adjacent to each other, and the calculation interval is reduced, as compared with a case wherein the plurality of transducers 122 are sequentially vibrated.

Therefore, when the calculation interval for the correlation calculation is reduced, construction of a motion-compensated ultrasonic image (e.g., a 4D elastographic image) is enabled by the 4D system. Further, since the amount of change in the tissue can be reduced when the calculation interval for the correlation calculation is small, the deviation of the cross section due to the motion (e.g., camera shaking by pressure or the body movements) can be reduced.

Moreover, in a case as shown in FIG. 4, wherein the plurality of transducers 122 are vibrated at the same time, the frame rate (or the volume rate) can be increased by (n+1) times, as compared with a case wherein the transducers are sequentially vibrated.

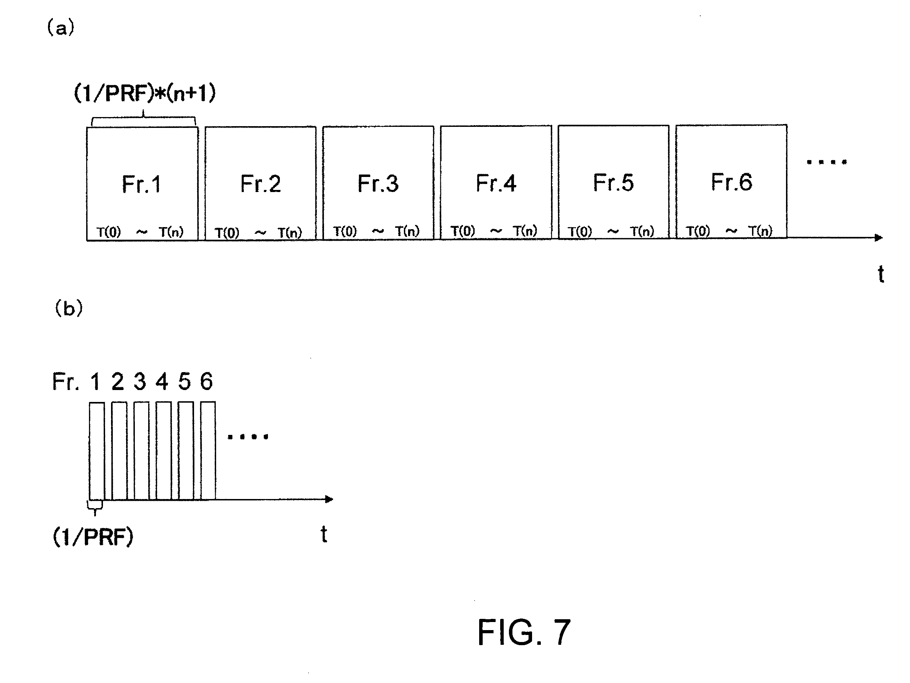

Next, the frame data acquisition sequence performed to create volume data (ultrasonic data) will now be described by reference to FIG. 7. In a case as shown in FIG. 7(a), wherein the plurality of transducers T(0) to T(n) are sequentially vibrated in the scanning sequence, a period of "(1/PRF)*(n + 1)" is required to obtain RF signal frame data for one cross section. Meanwhile, in a case in FIG. 7(b) wherein the plurality of transducers T(0) to T (n) are vibrated at the same time, a period of "(1/PRF)" is required to obtain RF signal frame data for one cross section. Therefore, in a case wherein RF signal frame data (ultrasonic data) for the cross section are generated by a single performance of irradiation of the plane wave (ultrasonic beam), frame data and volume data can be obtained in a short period of time, and the frame rate and the volume rate can be greatly increased, so that an image generation system that updates a three-dimensional image, as needed, and displays the updated image in real time can construct a motion-compensated 4D ultrasonic image.

As described above, the frame rate is improved for a case wherein the two-dimensional tomographic image configuration unit 113 creates a two-dimensional tomographic image (ultrasonic image) based on the RF signal frame data stored in the RF saving/selection unit 109. Therefore, the volume rate is improved for a case wherein, based on the two-dimensional tomographic image generated by the two-dimensional tomographic image configuration unit 113, the tomographic volume data generation unit 114 performs the three-dimensional coordinate transformation in accordance with the two-dimensional image acquisition location, and generates tomographic volume data (ultrasonic data). Furthermore, the volume rate is also improved for a case wherein the three-dimensional tomographic image configuration unit 115 performs volume rendering based on the luminance value and the opacity of the tomographic volume data, and generates a three-dimensional tomographic image (ultrasonic image).

Furthermore, the frame rate is also increased for a case wherein the two-dimensional elastographic image configuration unit 116 constructs a two-dimensional elastographic image (ultrasonic image) based on a plurality of RF signal frame data sets stored in the RF saving/selection unit 109. Therefore, the volume rate is increased for a case wherein, based on the two-dimensional elastographic image provided by the two-dimensional elastographic image configuration unit 116, the elastographic volume data generation unit 117 performs three-dimensional coordinate transformation in accordance with the two-dimensional elastographic image acquisition location, and generates elastographic volume data (ultrasonic data). Moreover, the volume rate is also increased for a case wherein the three-dimensional elastographic image configuration unit 118 performs volume rendering for the elasticity value and the opacity of the elastographic volume data, and generates a three-dimensional elastographic image (ultrasonic image).

Further, a period required by the synthesis unit 119 for synthesizing the two-dimensional tomographic image with the two-dimensional elastographic image, or synthesizing a three-dimensional tomographic image with a three-dimensional elastographic image, is also reduced.

The embodiment of the present invention has been described; however, the present invention is not limited to this embodiment, and can be varied or modified within the scope of the claims of the invention.

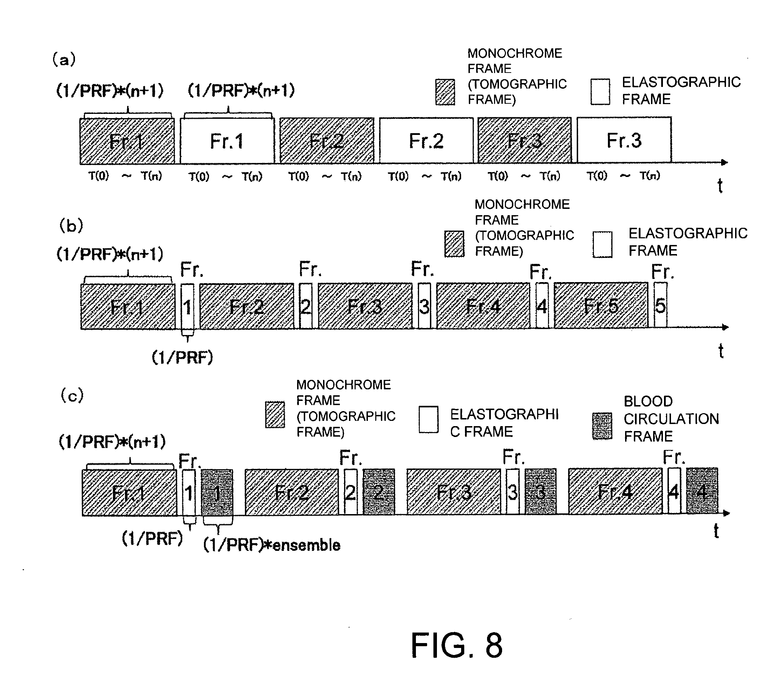

For example, in a case as shown in FIG. 8, wherein at least one ultrasonic image selected from among a tomographic image (monochrome image), an elastographic image (elastography), and a Doppler image (color Doppler image) is to be obtained, the plurality of transducers 122 may be vibrated at the same time to emit the ultrasonic beam to the subject 101, and in a case wherein an unselected ultrasonic image is to be obtained, the plurality of transducers 122 may be sequentially vibrated in the arrangement direction x, and emit the ultrasonic beam to the subject 101. According to this arrangement, for the selected ultrasonic image, the ultrasonic data for the cross section of the subject are generated by a single performance of irradiation of the ultrasonic beam, and therefore, the frame rate (or the volume rate) is increased, and the motion-compensated ultrasonic image can be created at a high frame rate or a high volume rate, while the unselected ultrasonic image can be constructed by performing focusing of the ultrasonic beam.

FIG. 8 (a) is a diagram showing alternate acquisition of tomographic frame data (RF signal frame data) for a tomographic image and elastographic frame data (RF signal frame data) for an elastographic image by sequentially vibrating the plurality of transducers T(0) to T(n). As shown in FIG. 8(a), in a case wherein the plurality of transducers T(0) to T(n) are sequentially vibrated, a period of "(1/PRF)*(n + 1) " is required for obtaining RF signal frame data (tomographic frame data and elastographic frame data) for a single cross section. In this case, both the tomographic volume image and the elastographic volume image can be constructed; however, as compared with a case wherein either the tomographic volume image or the elastographic image is to be constructed as a volume image, twice the period of time is required, and the volume rate is reduced to half.

FIG. 8(b) is a diagram showing the acquisition of tomographic frame data (RF signal frame data) for a tomographic image by sequential vibration of the plurality of transducers 122, and the acquisition of elastographic frame data (RF signal frame data) for an elastographic image by simultaneous vibration of the plurality of transducers T(0) to T(n). In this case, emission of the ultrasonic beam to the subject 101 is performed alternately by vibrating the plurality of transducers 122 at the same time and by vibrating the transducers 122 sequentially. Since the frame rate and the volume rate can be improved with this arrangement, a tomographic image, an elastographic image, and a Doppler image can be alternately scanned, and can be displayed by being superimposed with each other, and tomographic information, elastographic information, and blood circulation information can be displayed simultaneously in real time by a 4D system. It should be noted that the emission of the ultrasonic beam by simultaneous vibration and the emission of the ultrasonic beam by sequential vibration may be alternately performed for each emission of the ultrasonic beam, or may be alternately performed for every set of multiple emissions of the ultrasonic beam.

As shown in FIG. 8(b), a period of "(1/PRF)*(n + 1) " is required to obtain the tomographic frame data for a single cross section. Meanwhile, a period of "1/PRF" is required to obtain elastographic frame data for a single cross section. In this case, as compared with the case where both a tomographic volume image and an elastographic volume image are generated, the scanning period of time is reduced, and the volume rate is increased. Further, in a case wherein a tomographic image (ultrasonic image) is to be obtained by performing focusing (including multistep focusing) of the ultrasonic beam, the tomographic frame data (RF signal frame data) can be obtained by sequentially vibrating the plurality of transducers T(0) to T(n). That is, in a case wherein focusing of the ultrasonic beam is performed to obtain at least one ultrasonic image that is selected from a tomographic image, an elastographic image, and a Doppler image, ultrasonic data (RF signal frame data) can be obtained by sequentially vibrating the plurality of transducers 122.

Further, in a case wherein an elastographic image or a Doppler image is to be obtained, the ultrasonic beam may be emitted to the subject 101 by simultaneously vibrating the plurality of transducers 122 when the ultrasonic probe 102 is moved, or in a case wherein a tomographic image is to be obtained, the ultrasonic beam may be emitted to the subject 101 by sequentially vibrating the plurality of transducers 122 in the arrangement direction x when the ultrasonic probe 102 is moved. According to this arrangement, the scanning period is reduced and the volume rate is increased as compared with the case wherein a tomographic image, an elastographic image, and a Doppler image are to be constructed, and in a case wherein a tomographic image (ultrasonic image) is to be obtained, focusing of the ultrasonic beam is performed to obtain tomographic frame data.

FIG. 8(c) is a diagram showing the acquisition of elastographic frame data for an elastographic image and Doppler data (ultrasonic data) for a Doppler image by simultaneous vibration of the plurality of transducers T(0) to T(n).

For the conventional ultrasonic diagnostic device, it has been difficult to employ three or more ultrasonic images at the same time to display, in a 4D system, an observation mode that can be applied for a plurality of diagnoses, because the frame rate and the volume rate are reduced. According to this embodiment, however, the frame rate and the volume rate can be increased, and three or more ultrasonic images (e.g., a tomographic image, an elastographic image, and a blood circulation image) can be displayed by the 4D system.

In a case as shown in FIG. 8(c), wherein a tomographic image is to be obtained by performing focusing of the ultrasonic beam, the tomographic frame data (RF frame data) can be obtained by sequentially vibrating the plurality of transducers 122. Further, in a case wherein an elastographic image and a blood circulation image (Doppler image), for which focusing of the ultrasonic beam is not required, are to be obtained, the plane wave can be generated by vibrating the plurality of transducers 122 at the same time, and the elastographic frame data and the blood circulation data can be obtained. As a result, functional information (elastographic information and blood circulation information) can be displayed by a single scanning performance, while morphologic information (tomographic information) is maintained.

Since generally eight to ten sets of ensemble information are required to obtain a blood circulation image, and since the frame rate and the volume rate are reduced in a case wherein the plurality of transducers 122 are sequentially vibrated, it has been especially difficult to employ a 4D system to obtain a blood circulation image. According to this embodiment, however, since the frame rate and the volume rate can be increased, real-time display of the blood circulation information can be provided by the 4D system even in a case wherein about eight to ten sets of ensemble information are generally required, and the three-dimensional distribution of the stiffness of a tumor (elastographic information), for example, can be displayed in real time, and also, blood circulation information indicating either a pulsatile flow of blood or a stationary flow entering into the tumor can be displayed at the same time, to thereby provide a high value-added ultrasonic diagnostic device.

In the above described embodiment, the first ultrasonic data are not limited to even-numbered volume data, and the second ultrasonic data are not limited to odd-numbered volume data. In a case wherein, for example, the two-dimensional elastographic image configuration unit 116 constructs a two-dimensional elastographic image (ultrasonic image) based on a plurality of sets of RF signal frame data stored in the RF saving/selection unit 109, the RF signal frame data obtained at the same cross sectional location at different times may be employed as the first ultrasonic data and the second ultrasonic data. That is, the first ultrasonic data and the second ultrasonic data are not limited to volume data, and may be frame data.

Further, the first ultrasonic data and the second ultrasonic data are not limited to ultrasonic data obtained at the same cross sectional location, and may be ultrasonic data obtained at different cross sectional locations. For example, when the ultrasonic probe 102 is moved, the plurality of transducers 122 may be simultaneously vibrated to emit the ultrasonic beam to the subject 101 continuously multiple times (e. g. , three times or more), and the ultrasonic image generation unit 150 may perform a comparison for ultrasonic data (including the first ultrasonic data and the second ultrasonic data) that are generated based on continuous emission of the ultrasonic beam multiple times, and may generate an elastographic image based on the displacement of the subject 101 in the directions in three-dimensional space, (x, y, z).

As described above, the plurality of transducers are vibrated at the same time, and emit the ultrasonic beam to the subject 101 continuously multiple times. According to this arrangement, when the ultrasonic data generated based on continuous emission of the ultrasonic beams multiple times are compared with each other, the ultrasonic image can be generated based on the displacement of the subject in the directions in three-dimensional space, (x, y, z).

Furthermore, when the plurality of transducers are moved, the plurality of transducers are vibrated simultaneously to emit the ultrasonic beam to the subject 101 continuously multiple times, and the ultrasonic image generation unit 150 generates an elastographic image based on the ultrasonic data that have been generated by continuous emission of the ultrasonic beams multiple times. According to this arrangement, when the ultrasonic data generated by continuous emission of the ultrasonic beam multiple times are compared with each other, the elastographic image can be generated based on the displacement of the subject 101 in the directions in three-dimensional space (x, y, z), and the scanning period for one frame can be reduced, so that the correlation accuracy can be improved.

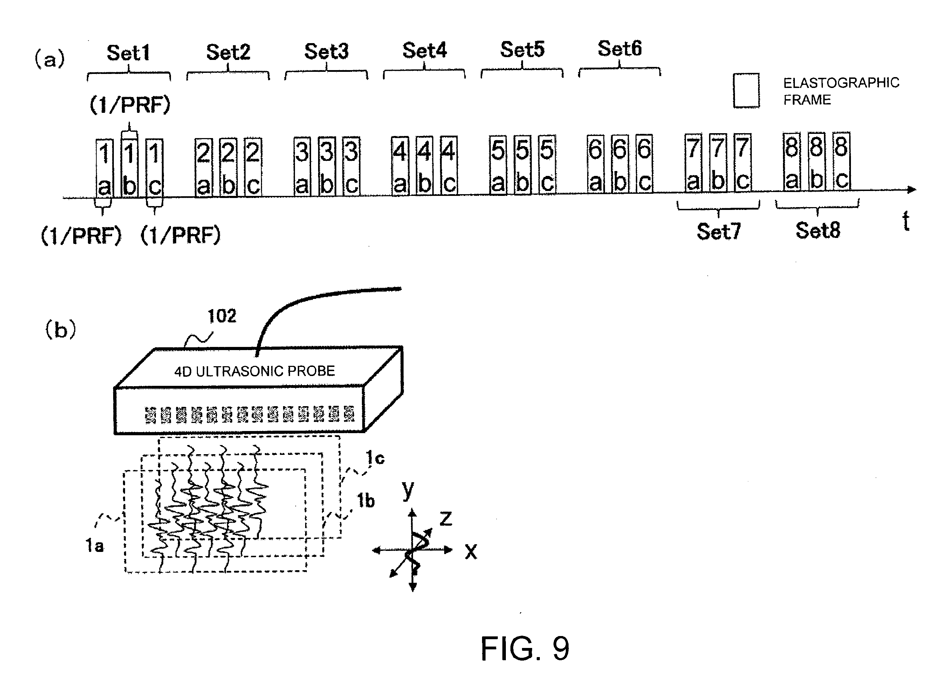

An explanation will be given by reference to FIG. 9 for a case wherein an elastographic image is to be generated based on the displacement in the directions in three-dimensional space (x, y, z). In the above-described embodiment, two ultrasonic images are compared, and the tissue displacement due to the body motion or the external pressure is employed to generate elastographic information or an elastographic image. As a result, the displacement of the cross section parallel to the ultrasonic probe 102 or the ultrasonic transmission/reception face can be measured.

That is, the displacement of the cross section in the arrangement direction x of the transducers 122 and parallel to a direction y, which is perpendicular to the arrangement direction x, can be measured. On the other hand, as shown in FIG. 9, when the plurality of transducers 122 are vibrated at the same time, and transmission and reception of the plane wave are repeated three times, continuous elastographic frame data (e.g., elastographic frame data 1a, 1b, and 1c) are obtained as one set of data, and as a result, the displacement not only in the arrangement direction x and in the vertical direction y, but also the displacement in the short axial direction z can be measured. The plurality of transducers 122 perform irradiation of the ultrasonic beam continuously three times or more. With this arrangement, when the ultrasonic data generated by continuous emission of the ultrasonic beams three times are compared with each other, the elastographic image can be generated based on the displacement of the subject in the directions in three-dimensional space, (x, y, z).

For the conventional ultrasonic diagnostic device, difficulty is encountered in obtaining ultrasonic data (RF signal frame data) for the cross section continuously multiple times, because the frame rate and the volume rate are reduced. According to this embodiment, however, since the frame rate and the volume rate can be increased, emission of the ultrasonic beam to the subject is repeated sequentially multiple times (e.g., three times or more) by simultaneously vibrating the plurality of transducers 122, and the continuous elastographic frame data (ultrasonic data) are created, so that the correlation or the search can be performed in the directions in three-dimensional space (x, y, z), including the short axial direction z. As a result, even in a case wherein a small child who does not hold still, or an animal, is a subject, the 4D system can be employed to display an elastographic image in real time. Furthermore, since the scanning period for one frame is reduced by transmission/reception of the plane wave, the correlation accuracy can be improved.

Industrial Applicability

The ultrasonic diagnostic device of the present invention provides the effect that the frame rate or the volume rate is increased, and is useful as an ultrasonic diagnostic device that can construct a motion-compensated ultrasonic image (e.g., a 4D ultrasonic image) at a high frame rate or a high volume rate.

Reference Signs List

| | |
|---|---|
| 100: | ultrasonic diagnostic device |
| 101: | subject |
| 102: | ultrasonic probe |
| 103: | control unit |
| 104: | operation unit |
| 105: | transmission unit |
| 106: | reception unit |
| 107: | ultrasonic wave transmission/reception control unit |
| 108: | phase-rectification/addition unit |
| 109: | RF saving/selection unit |
| 111: | data storage unit |
| 113: | two-dimensional tomographic image configuration unit |
| 114: | tomographic volume data generation unit |
| 115: | three-dimensional tomographic image configuration unit |
| 116: | two-dimensional elastographic image configuration unit |
| 117: | elastographic volume data generation unit |
| 118: | three-dimensional elastographic image configuration unit |
| 119: | synthesis processing unit |
| 120: | display unit |
| 122: | transducer |
| 150: | ultrasonic image generation unit |

**Claims**

1. An ultrasonic diagnostic device, **characterized by** comprising:

   a plurality of transducers arranged in an ultrasonic probe so as to be vibrated at the same time for projecting an ultrasonic beam to a subject; and
   an ultrasonic image generation unit for generating first ultrasonic data through projection of a first ultrasonic beam to a subject by vibrating the plurality of transducers at the same time, and generating second ultrasonic data through projection of a second ultrasonic beam to the subject by vibrating the plurality of transducers at the same time, and for generating an elastographic image based on the first ultrasonic data and the second ultrasonic data.

2. The ultrasonic diagnostic device according to claim 1, **characterized in that**:

   the plurality of transducers of the ultrasonic probe perform forward and backward movements; and
   the ultrasonic image generation unit generates the elastographic image based on the first ultrasonic data, generated during the forward movement, and the second ultrasonic data generated during the backward movement, which is performed immediately after the forward movement.

3. The ultrasonic diagnostic device according to claim 1, **characterized in that** the ultrasonic image generation unit includes an ultrasonic image generator that generates a three-dimensional ultrasonic image based on the first ultrasonic data and the second ultrasonic data.

4. The ultrasonic diagnostic device according to claim 1, **characterized in that** the plurality of transducers encourage generation of plane waves.

5. The ultrasonic diagnostic device according to claim 1, **characterized in that**:

   in a case wherein at least one ultrasonic image selected from a tomographic image, an elastographic image, and a Doppler image is to be obtained, the plurality of transducers are vibrated at the same time to project the ultrasonic beam to the subject; and
   in a case wherein an ultrasonic image that is not selected is to be obtained, the plurality of transducers are sequentially vibrated in the arrangement direction to project the ultrasonic beam to the subject.

6. The ultrasonic diagnostic device according to claim 1, **characterized in that**:

   in a case wherein an elastographic image or a Doppler image is to be obtained, when the plurality of transducers are moved, the plurality of transducers are vibrated at the same time to project the ultrasonic beam to the subject; and
   in a case wherein a tomographic image is to be obtained, when the plurality of transducers are moved, the plurality of transducers are vibrated sequentially in the arrangement direction to project the ultrasonic beam to the subject.

7. The ultrasonic diagnostic device according to claim 1, **characterized in that** the ultrasonic beam generated by vibrating the plurality of transducers at the same time, and the ultrasonic beam generated by sequentially vibrating the plurality of the transducers in the arrangement direction are alternately projected to the subject.

8. The ultrasonic diagnostic device according to claim 1, **characterized in that** the ultrasonic beam generated by vibrating the plurality of transducers at the same time is projected to the subject continuously multiple times.

9. The ultrasonic diagnostic device according to claim 1, **characterized in that**:

   when the plurality of transducers are moved, the plurality of transducers are vibrated at the same time, and project the ultrasonic beam to the subject continuously multiple times; and
   the ultrasonic image generation unit generates an elastographic image by employing ultrasonic data that are generated by continuous irradiation of the ultrasonic beam multiple times.

10. The ultrasonic diagnostic device according to claim 9, **characterized in that** the plurality of transducers perform irradiation of the ultrasonic beam continuously three times or more.

**11.** The ultrasonic diagnostic device according to claim 1, **characterized in that**:

the plurality of transducers of the ultrasonic probe perform forward and backward movements; and
the ultrasonic image generation unit generates the elastographic image based on the first ultrasonic data generated during the forward or backward movement and the second ultrasonic data generated during the same movement as the forward or backward movement.

**12.** The ultrasonic diagnostic device according to claim 11, **characterized in that** the first ultrasonic data and the second ultrasonic data are ultrasonic data for cross sections adjacent to each other.

**13.** The ultrasonic diagnostic device according to claim 11, **characterized by** comprising:

a plurality of transducers arranged in the ultrasonic probe so as to be vibrated at the same time for projecting an ultrasonic beam to a subject; and
an ultrasonic image generation unit for generating a three-dimensional ultrasonic image based on ultrasonic data obtained when the plurality of transducers are moved.

**14.** An ultrasonic image generation method **characterized by** comprising the steps of:

projecting, to a subject, an ultrasonic beam generated by vibrating, at the same time, a plurality of transducers arranged in an ultrasonic probe;
generating first ultrasonic data through projection of a first ultrasonic beam to the subject by vibrating the plurality of transducers at the same time;
generating second ultrasonic data through projection of a second ultrasonic beam to the subject by vibrating the plurality of transducers at the same time; and
generating an ultrasonic image based on the first ultrasonic data and the second ultrasonic data.

FIG.1

(a)

SIMULTANEOUS
TRANSMISSION FROM
INDIVIDUAL
TRANSDUCERS →

122

X

GENERATION OF
PLANE WAVE →

TRANSMISSION

FRAME RATE
1000 – 10000 [fps]

(b)

RECEPTION

REFLECTED
SIGNAL ←

(c)

RECEPTION/PHASE-
RECTIFICATION

FIG. 2

(a)

Ve(0)  Ve(50)  Ve(m)

M1

Vo(0)

Vo(50)  M2

Vo(m)

EVEN-NUMBERED
VOLUME DATA

ODD-NUMBERED
VOLUME DATA

(b)

M1

TRANSDUCER T(n)

x

TRANSDUCER T(0)

z

101

y

Ve(0)  Ve(50)  Ve(m)

——— BEAM TRANSMISSION
     SEQUENCE

⟹ SWING DIRECTION

(c)

M2

z

101

TRANSDUCER T(n)

x

TRANSDUCER T(0)

y

Vo(0)  Vo(50)  Vo(m)

——— BEAM TRANSMISSION
     SEQUENCE

⟹ SWING DIRECTION

FIG.3

FIG. 4

EP 2 781 192 A1

FIG. 5

F I G . 6

(a)

(1/PRF)*(n+1)

| Fr.1 | Fr.2 | Fr.3 | Fr.4 | Fr.5 | Fr.6 | .... |
|------|------|------|------|------|------|------|
| T(0) ~ T(n) | T(0) ~ T(n) | T(0) ~ T(n) | T(0) ~ T(n) | T(0) ~ T(n) | T(0) ~ T(n) | |

t

(b)

Fr. 1 2 3 4 5 6

....

(1/PRF)

t

FIG. 7

EP 2 781 192 A1

FIG. 8

FIG. 9

## INTERNATIONAL SEARCH REPORT

| | |
|---|---|
| | International application No. |
| | PCT/JP2012/075746 |

| A. CLASSIFICATION OF SUBJECT MATTER |
|---|
| *A61B8/08*(2006.01)i |
| According to International Patent Classification (IPC) or to both national classification and IPC |

| B. FIELDS SEARCHED |
|---|
| Minimum documentation searched (classification system followed by classification symbols) |
| A61B8/08 |

| Documentation searched other than minimum documentation to the extent that such documents are included in the fields searched |
|---|
| Jitsuyo Shinan Koho          1922–1996    Jitsuyo Shinan Toroku Koho    1996–2012 |
| Kokai Jitsuyo Shinan Koho    1971–2012    Toroku Jitsuyo Shinan Koho    1994–2012 |

| Electronic data base consulted during the international search (name of data base and, where practicable, search terms used) |
|---|
| |

| C. DOCUMENTS CONSIDERED TO BE RELEVANT |
|---|

| Category* | Citation of document, with indication, where appropriate, of the relevant passages | Relevant to claim No. |
|---|---|---|
| X<br>Y<br>A | JP 2009-201989 A  (Siemens Medical Solutions USA, Inc.),<br>10 September 2009 (10.09.2009),<br>paragraphs [0002], [0020], [0032] to [0034], [0050], [0051]<br>(Family: none) | 1,3,4,8-10, 14<br>11-13<br>2 |
| Y | WO 2011/30812 A1  (Hitachi Medical Corp.),<br>17 March 2011 (17.03.2011),<br>paragraphs [0072], [0076]; fig. 3<br>& EP 2476376 A1          & CN 102481145 A | 11-13 |
| A | JP 2004-222798 A  (Hitachi Medical Corp.),<br>12 August 2004 (12.08.2004),<br>paragraphs [0027] to [0029]<br>(Family: none) | 2 |

| ☒  Further documents are listed in the continuation of Box C. | ☐  See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 18 December, 2012 (18.12.12) | 25 December, 2012 (25.12.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.

PCT/JP2012/075746

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2009-28366 A  (Toshiba Corp.), 12 February 2009 (12.02.2009), claim 8; fig. 6, 8, 13 & US 2009/0043209 A1    & CN 101352354 A | 2 |
| A | JP 2010-246692 A  (Furuno Electric Co., Ltd.), 04 November 2010 (04.11.2010), paragraph [0079]; fig. 5(a) & US 2010/0257935 A1    & EP 2241260 A1 & CN 101865931 A         & AT 533406 T | 1-4,8-14 |

Form PCT/ISA/210 (continuation of second sheet) (July 2009)

## INTERNATIONAL SEARCH REPORT

| International application No. |
|---|
| PCT/JP2012/075746 |

**Box No. II          Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III          Observations where unity of invention is lacking (Continuation of item 3 of first sheet)**

This International Searching Authority found multiple inventions in this international application, as follows:

Document (JP 2009-201989 A) discloses an ultrasonic wave image generating device for generating an elastic image by means of a plane wave (paragraphs [0020] and [0050]).

Additionally, generating a tomographic image on the basis of first and second ultrasonic wave data is also suggested (paragraphs [0020], [0032] to [0034] and [0050]).

Here, generating a plane wave by vibrating a plurality of transducers simultaneously is a known art.

(Continued to extra sheet)

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
   1-4 and 8-14

**Remark on Protest**          ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2012/075746

Continuation of Box No.III of continuation of first sheet(2)

Therefore, the invention of claim 1 cannot be considered to be novel in the light of the invention disclosed in JP 2009-201989 A, and does not have a special technical feature.

Consequently, two inventions (invention groups) each having a special technical feature indicated below are involved in claims.

Meanwhile, the inventions of claims 1, 3, 4, 8-10 and 14 having no special technical feature are classified into invention 1.

(Invention 1) the inventions of claims 1-4 and 8-14

An invention pertaining to a constitution in which a plurality of transducers perform reciprocating motions in an ultrasonic wave image generating device for generating an elastic image.

(Invention 2) the inventions of claims 5-7

An invention pertaining to a constitution in which a plurality of transducers perform a simultaneous vibration and a sequential vibration in an ultrasonic wave image generating device for generating an elastic image.

Form PCT/ISA/210 (extra sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004141505 A

- JP 2008259555 A